# EUROPÄISCHE PATENTSCHRIFT

(11) **EP 2 512 457 B1**
(45) Veröffentlichungstag und Bekanntmachung des Hinweises auf die Patenterteilung: **28.10.2015**
(21) Anmeldenummer: 10790956.6
(22) Anmeldetag: 14.12.2010
(51) Int. Cl.: A61K 9/32

(54) **FILMÜBERZUGSMITTEL BASIEREND AUF POLYVINYLALKOHOL-POLYETHER-PFROPFCOPOLYMEREN- POLYVINYLALKOHOL-KOMBINATIONEN MIT VERBESSERTER FEUCHTIGKEITSBARRIEREWIRKUNG**
FILM COATING AGENTS BASED ON COMBINATIONS OF POLYVINYL ALCOHOL-POLYETHER GRAFT POLYMERS AND POLYVINYL ALCOHOL, HAVING AN IMPROVED MOISTURE BARRIER EFFECT
AGENT DE PELLICULAGE À BASE D'ASSOCIATIONS D'ALCOOL DE POLYVINYLE ET DE COPOLYMÈRES GREFFÉS D'ALCOOL DE POLYVINYLE ET DE POLYÉTHER, À EFFET BARRIÈRE AMÉLIORÉ VIS-À-VIS DE L'HUMIDITÉ

(30) Priorität: 16.12.2009 EP 09179506
(43) Veröffentlichungstag der Anmeldung: 24.10.2012
(73) Patentinhaber: BASF SE, 67056 Ludwigshafen (DE)
(72) Erfinder: CECH, Thorsten, 68647 Biblis (DE); GEISELHART, Verena, 68542 Heddesheim (DE); AGNESE, Thorsten, 67069 Ludwigshafen (DE)
(86) Internationale Anmeldenummer: PCT/EP2010/069556
(87) Internationale Veröffentlichungsnummer: WO 2011/082980

(56) Entgegenhaltungen:
- EP-A1- 2 106 789
- WO-A2-2006/002808

## Beschreibung

Die vorliegende Erfindung betrifft Filmüberzüge zum Überziehen von Dosierungsformen von pharmazeutischen oder diätetischen Wirkstoffen oder Nahrungsergänzungsmitteln, wobei die Filmüberzugsmittel aus einer co-prozessierten Mischung von Polyvinylalkohol-Polyether-Pfropfcopolymer und Polyvinylalkohol (Komponente A) und einem Vinylpyrrolidon-Vinylacetat-Copolymeren (Komponente B), organischen oder anorganischen Pigmenten (Komponenten C), gegebenenfalls einem Tensid, vorzugsweise einem Tensid mit einem HLB-Wert größer 10 (Komponente D) und weiteren üblichen Coatingbestandteilen bestehen. Die Filmüberzüge sollen vorzugsweise für Instant-Release-Überzüge eingesetzt werden.

Feste Darreichungsformen werden aus den verschiedensten Gründen mit einem schnelllöslichen Überzug versehen. So kann beispielsweise das Aussehen, die Unterscheidbarkeit und die Schluckbarkeit verbessert werden, ein bitterer Geschmack überdeckt werden oder die Darreichungsform gegenüber äußeren Einflüssen wie z. B. Feuchtigkeit oder Sauerstoff geschützt werden. Da sich der Filmüberzug schnell in verschiedenen wässrigen Medien u. a. in künstlichem Magen- und Darmsaft lösen soll, muss der wichtigste Bestandteil der Coatingzubereitung ein wasserlösliches, filmbildendes Polymer sein. Zum Überziehen von Tabletten werden als filmbildende Polymere hauptsächlich Hydroxypropylmethylcellulose und Hydroxypropylcellulose eingesetzt, die jedoch gravierende Nachteile aufweisen. So ist die Viskosität dieser Polymere in Wasser sehr hoch und erlaubt nur eine Konzentration bis ca. 10 %, da aufgrund der hohen Viskosität bei höheren Konzentrationen keine feine Zerstäubung in der Sprühdüse mehr möglich ist und der Überzug rauh, inhomogen und unansehnlich wird. Weiterhin sind diese Polymere sehr spröde und erleiden häufig Risse während der Lagerung, insbesondere wenn der Kern durch Feuchtigkeitsaufnahme oder -abgabe sein Volumen verändert.

Die Verwendung von Polyvinylalkohol-Polyether-Pfropfcopolymeren als Überzugsmittel oder Bindemittel in pharmazeutischen Darreichungsformen oder als Verpackungsmaterial oder als Zusatzstoff in kosmetischen, dermatologischen oder hygienischen Zubereitungen ist beispielsweise aus der WO 00/18375 bekannt. So wird beispielsweise eine Rezeptur für ein Filmüberzugsmittel beschrieben, welches aus einem Polyvinylalkohol-Polyether-Pfropfcopolymer und den üblichen Coatingbestandteilen zum Färben und Decken, nämlich Eisenoxid, Talkum und Titandioxid besteht. Ein so gearteter Überzug ist zwar flexibel, jedoch verhältnismäßig weich und zeigt, wenn Scherkräfte auf ihn einwirken, Abriebserscheinungen. Dies spielt besonders eine Rolle bei sehr großen Coatingansätzen, weil dann der hohe Druck bedingt durch die hohe Schüttung der Tabletten in Verbindung mit der rollierenden Bewegung der Tabletten in der Trommel entsprechend hohe Scherkräfte erzeugt. Da viele Arzneistoffe und auch einige Hilfsstoffe sehr lipophil sind, haften die Überzüge häufig schlecht auf der Tablettenoberfläche. Darüber hinaus sind Glätte und Glanz von solchen Filmüberzugsmitteln häufig nicht zufriedenstellend.

Polyvinylalkohol ist ebenfalls als Filmbildner bekannt, wird aber wegen verschiedener Nachteile seltener eingesetzt. Die Verwendung von polyvinylalkoholhaltigen Zubereitungen, die aus Polyvinylalkohol, Weichmacher und Talkum bestehen, wird in WO 01/04195 beschrieben. Nachteilig bei diesen Zubereitungen sind die langsame Auflösung bei der Herstellung der wässrigen Coatinglösung, die hohe Viskosität, die niedrige Konzentration in der Sprühlösung, die Verwendung von Weichmachern und die langsame Auflösungsgeschwindigkeit des Filmüberzuges, insbesondere nach Lagerung sowie eine Versprödung des Filmüberzuges nach Lagerung einhergehend mit Rissbildungen. Zudem kommt es bei der Versprühung von höherkonzentrierten Polyvinylalkohollösungen (> 8%) zu Fadenbildung an der Sprühdüse.

Die in der Pharmakopoea auch als Copolyvidon oder Copovidon bezeichneten Copolymere erhalten aus N-Vinylpyrrolidon und Vinylacetat im Gewichtsverhältnis 60:40 ist ebenfalls als Filmbildner bekannt. Da die Wasseraufnahme von Copovidon vergleichsweise hoch ist, ist Copovidon allein als Filmbildner selten geeignet und wird üblicherweise mit weniger hygroskopischen Substanzen kombiniert um die Sprödigkeit und Löslichkeit der Filmüberzüge zu verbessern. Es wird auch erwähnt, dass Copovidon als Subcoating eingesetzt werden kann um Tablettenkernen mit feuchtigkeitsempfindlichen Wirkstoffen vor dem Auftragen von wässrigen Dragierlösungen oder Filmüberzügen zu schützen. (Vgl. V. Bühler, "Polyvinylpyrrolidone Excipients for Pharmaceuticals",S. 214-216, Springer Verlag Berlin Heidelberg 2005). Allerdings kann Copovidon allein nur aus organischer Lösung als Zwischenschicht aufgebracht werden.

Pigmente sind ein wesentlicher Bestandteil von Filmüberzügen. Sie dienen im Filmüberzug nicht nur farbgebenden Zwecken, sondern können auch eine gewisse Barrierewirkung entfalten. Allerdings sind höhere Pigmentgehalte im Filmüberzugsmittel anwendungstechnisch nachteilig, da zum einen die Einarbeitung höherer Pigmentanteile in eine wässrige Sprühsuspension aufwendig ist, zum anderen auch das Versprühen hochpigmenthaltiger Suspensionen schwerer zu steuern ist. Hinzu kommt, dass hochpigmenthaltige Sprühsuspensionen eher zur Entmischung neigen.

In der WO 03/070224 werden Überzüge beschrieben, die aus Polyvinylalkohol-Polyether-Pfropfcopolymeren, einer Komponente mit Hydroxy-, Amid- oder Esterfunktionen und weiteren üblichen Coatingbestandteilen bestehen. Dabei wird zunächst ein Prämix der Einsatzstoffe als physikalische Mischung hergestellt und diese anschließend in Wasser dispergiert. Diese Zubereitungen neigen zur Entmischung und weisen keine guten Rauhigkeitswerte auf.

In der WO 2006/002808 werden schnelldispergierbare Filmüberzugsmittel basierend auf Polyvinylakohol-Polyether-Pfropfpolymeren beschrieben, die aufgrund besonders feinteiliger Pigmente eine niedrige Rauhigkeit und gute physikalische Stabilität aufweisen. Beschrieben werden auch Kombinationen zweier Polymere wie beispielsweise eine Zweier-Kombination des Pfropfpolymeren mit einem Vinylpyrrolidon-Vinylacetat-6:4-Copolymer. Allerdings sind solchen Überzugsmitteln bei der Pigmentbeladung grenzen gesetzt. Auch die Wasserdampfpermeation ist bei solchen Filmen noch nicht befriedigend.
Grundsätzlich sind Filmüberzüge mit einer hohen Pigmentbeladung von möglichst 50 Gew.-% oder mehr wünschenswert. Bisher bekannte Filmüberzugsmittel mit höherer Pigmentbeladung erfüllen jedoch die Anforderungen an die Wasserdampfpermeation nicht.

Aufgabe der Erfindung war es, verbesserte Filmüberzugsmittel zu finden, die einen verbesserten Feuchtigkeitsschutz für den Wirkstoff aufweisen. Solche Filmüberzugsmittel sollen möglichst schnell in Wasser auflösbar sein und so für Instant-Release-Formen geeignet sein. Ausserdem sollen die Filmüberzüge hinsichtlich ihrer Pigmentbeladung optimiert sein. Die Überzugsmittel sollen auch in Pulverform zu keinerlei Entmischung zwischen den einzelnen Bestandteilen neigen, insbesondere nicht zur entsmischung zwischen Pigmenten und Polymeren, und sollen weiterhin ausgezeichnet fließfähig sein.

Demgemäß wurden Filmüberzugsmittel basierend auf einer co-prozessierten Mischung aus einem Polyvinylalkohol-PolyetherPfropfpolymeren und Polyvinylalkohol (Komponente A), einem N-Vinylpyrrolidon-Vinylacetat-Copolymer mit einem Gewichtsverhältnis 6:4 (Komponente B), Pigmenten (Komponente C), gegebenenfalls Tensiden (Komponente D) und gegebenenfalls weiteren pharmazeutischen Hilfssstoffen (Komponenten E), gefunden.

Die Filmüberzugsmittel können wie folgt zusammengesetzt sein:
A) 20-45 Gew.% einer co-prozessierten Mischung aus Polyvinylalkohol-Polyether-Pfropfcopolymeren und Polyvinylalkohol (Komponente A),
B) 5- 20 Gew.-% eines Polyvinylpyrrolidons oder eines Vinylpyrrolidon-Vinylacetat-Copolymeren(Komponente B)
C) 20-75 Gew.% Pigmente (Komponente C)
D) 0 - 10 Gew.-% eines oder mehrerer Tenside (Komponente D) sowie
E) 0 - 30 Gew.-% weiteren üblichen Coatingbestandteilen (Komponenten E),
wobei sich die Menge der Komponenten A bis E zu 100 Gew.-% addiert.

Die Komponente A stellt eine co-prozessierte Mischung aus einem Polyvinylalkohol-Polyether-Pfropfcopolymeren und Polyvinylalkohol dar.

Unter Polyvinylalkohol-Polyether-Pfropfcopolymeren werden Polymere verstanden, die erhältlich sind durch Polymerisation von mindestens einem Vinylester von aliphati sehen C₁-C₂₄-Carbonsäuren bevorzugt Vinylacetat, in Gegenwart von Polyethem als Pfropfgrundlage und anschließende vollständige oder teilweise Verseifung der Polyvinylestergruppen.

Bevorzugt sind Polyether mit einem mittleren Molgewicht zwischen 400 und 50000 g/mol, besonders bevorzugt 1500 bis 20000 g/mol.

Die Herstellung solcher Pfropfcopolymere ist an sich bekannt.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

Bevorzugt sind Polymere mit einem Verseifungsgrad der Polyvinylestergruppen von > 70 mol-%, besonders bevorzugt > 80 mol-% und ganz besonders bevorzugt von > 85 mol-%.

Besonders bevorzugt ist ein Polyvinylalkohol-Polyether-Pfropfcopolymer bei dem Vinylacetat als zu pfropfendes Monomer und Polyethylenglykol 6000 als Pfropfgrundlage verwendet wurde, und der Verseifungsgrad der Estergruppen > 85 mol-% liegt, wobei das Massenverhältnis der Molekülteile Polyvinylalkohol / Polyethylenglykol 6000 bei 75:25 liegt.

Unter Polyvinylalkohol wird ein durch Homopolymerisat verstanden, das üblicherweise durch Polymerisation von Vinlyacetat und Verseifung der Acetatgruppen erhalten wird. Geeignete Polyvinylalkohole weisen vorzugsweise eine Hydrolysegrad (Verseifungsgrad der Acetatgruppe) von > 80 mol-%, bevorzugt > 85 mol-% auf. Die Viskositäten einer 4 gew.-%igen wässrigen Lösung bei 20 °C können 3 bis 30, bevorzugt 3 bis 20 mPas betragen.

Pfropfpolymer und Polyvinylalkohol werden co-prozessiert, sodass eine innige Mischung der Einzelkomponenten vorliegt,. Die Einzelkomponenten sind aufgrund des Herstellverfahrens ineinander eingebettet, sodass sie sich nicht entmischen und die Einbettung mit mechanischen Mitteln nicht mehr getrennt werden kann. Die Herstellung der Komponente A erfolgt durch gemeinsames Versprühen einer wässrigen Lösung der Polymeren.
Vorzugsweise enthält die Komponente A auch Siliciumdioxid. Eine besonders bevorzugte Komponente A besteht aus 55 -65 Gew.-% Pfropfpolymer, 35 bis 45 Gew.-% Polyvinylalkohol und 0.1 bis 0.3 Gew.-% Silicumdioxid.

Die Komponente A ist in Mengen von 20 bis 45 Gew.-% enthalten.

Weiterhin enthalten die Filmüberzugsmittel als Komponenten B ein aus den Pharmakopöen als Copolyvidon oder Copovidon bekanntes Copolymer, das aus Vinylpyrrolidon und Vinylacetat im Gewichtsverhältnis 60:40 erhalten wird. Der K-Wert nach Fikentscher, gemessen 1 gew.-%ig in Wasser, dieser Polymeren liegt im Bereich von 20 bis 50, bevorzugt 25 bis 35. Die Komponenten B sind in Mengen von 5 bis 20 Gew.-% enthalten

Weiterhin enthalten die Filmüberzugsmittel als Komponenten C organische oder anorganische Pigmente.

Als Pigmente werden farbgebende oder weiße Substanzen bezeichnet, die im Anwendungsmedium nicht löslich sind. Die Pigmente können anorganische oder organische Pigmente sein.

Als anorganische Pigmente eignen sich Aluminiumsilikate, Magnesiumsilikate, Magnesium-aluminiumsilikate, Eisenoxid, Titandioxid, Zinkoxid, Kieselsäure, oder Calciumhydrogenphosphat. Von den Aluminiumsilikaten kommt vor allem Kaolin in Betracht. Von den Magnesiumsilikaten hat vor allem Talkum Bedeutung.
Bevorzugte Pigmente sind Eisenoxid und Weißpigmente ausgewählt aus der Gruppe bestehend aus Titandioxid, Talkum und Kaolin.

Als organische Pigmente eignen sich organische Farblacke oder Mischungen davon. Als organische Farblacke können z.B. verwendet werden: Chinolingelblack, Tartrazinlack, Gelborangelack, FD&C Gelb-Aluminiumlack, Cochenillerotlack, Erythrosinlack, Azorubinlack, Indigotinlack, Betacarotin.

Die Pigmente sind vorzugsweise in Mengen von 20 bis 75 Gew.-% enthalten.

Als Komponenten D können gewünschtenfalls Tenside eingesetzt werden, vorzugsweise Tenside mit einem HLB-Wert größer 10 (HLB-Wert: Hydrophilic Lipophilic Balance; Vgl. Fiedler, Lexikon der Hilfsstoffe, Editio Cantor Verlag Aulendorf, 5.Auflage (2002), Seite 115-121) größer 10 eingesetzt werden. Wenn Tenside zum Einsatz kommen, so können sie in Mengen von 0.1 bis 10 Gew.-%, vorzugsweise 1 bis 5 Gew.-%, eingesetzt werden.

Besonders geeignet sind Alkalisalze von C8-C30-Fettsäuren , C8-C30-.Alkylsulfonaten, C8-C30-Alkylsulfaten, C8-C30-Alkylarylsulfonaten oder Dioctylsulfosuccinat, Ethoxylate von C8-C30-Fettsäuren, C8-C30-Fettalkoholen, Fettsäureglyceriden, Sorbitanfettsäureestern, Sorbitanfettalkoholethern oder Phenolen, sowie Polyoxypropylen-Polyoxyethylen-Blockcopolymeren. Beispiele aus den genannten Stoffklassen sind Natriumstearat, Natriumoleat, Natriumlaurylsulfonat, Natriumlaurylsulfat, Polyoxyethylen(9)monostearat, Polyoxyethylen(10)stearylcetylether, Polysorbat 80, Polysorbat 20, ethoxyliertes Ricinusöl (35 EO), ethoxyliertes hydriertes Ricinusöl (40 EO), ethoxylierte 12-Hydroxystearinsäure (15 EO), Poloxamer 188, Poloxamer 408.

Weiterhin können die Filmüberzüge als Komponenten E zusätzliche Hilfsstoffe enthalten, wie sie als Coatingbestandteile üblich sind. Weitere übliche Coatingbestandteile umfassen:
wasserlösliche Farbstoffe, Antiklebemittel, Füllstoffe, Glanzverstärker, Schaumverhinderer, Schutzkolloide, Puffersubstanzen, pH-regulierenden Substanzen oder Haftvermittler.

Die erfindungsgemäßen Filmüberzugsmittel können zunächst als weiße Zubereitung hergestellt werden, denen später weitere pulverförmige farbgebende Komponenten zugesetzt werden können. Die positiven Eigenschaften der Filmüberzüge bleiben auch bei dieser Vorgehensweise erhalten.

Die erfindungsgemäßen Zubereitungen bedürfen üblicherweise keines Weichmachers. Weichmacherfreiheit ist ein enormer Vorteil, weil Weichmacher oft zu Problemen bei der Lagerung von überzogenen Formen führen. So kann der Weichmacher in den Kern migrieren und den Wirkstoff in seinen physikalischen und chemischen Eigenschaften verändern, der Film versprödet dadurch und neigt zur Rissbildung. Die meisten Weichmacher weisen zudem eine gewisse Flüchtigkeit auf, die ebenfalls zu einer Versprödung führt. Allerdings ist gewünschtenfalls ein Zusatz von Weichmacher möglich.

Die Herstellung der Filmüberzugsmittel kann zum Beispiel erfolgen, indem zunächst die Komponenten A und B in Wasser aufgelöst werden. Diese Lösung kann anschliessend mit einer wässrigen Pigmentsuspension vereinigt werden. Die Pigmentsuspension kann erhalten werden, indem die Pigmente, gegebenenfalls unter Zusatz von oberflächenaktiven Substanzen, in Wasser homogenisiert werden.

Weiterhin können auch alle Einsatzstoffe gemeinsam in Wasser eingebracht und homogenisiert werden. Bei dieser Vorgehensweise kann sich auch der Einsatz eines Antischaumsmittels empfehlen.

Die Menge an eingesetzten Wasser wird bei den geschilderten Verfahrensweisen so gewählt, dass die erhaltenen wässrigen Dispersionen der erfindungsgemäßen Filmüberzugsmittel Feststoffgehalte von 5 bis 45, bevorzugt 15 bis 30 Gew.-%, aufweisen.

Die wässrigen Filmüberzugsmittel können auch in Trockenpulver überführt werden. Die Trocknung der wässrigen Dispersionen kann in einem Sprühtrockner, Schaufeltrockner oder Wirbelschichttrockner erfolgen. In der Regel wird die zu trocknende Dispersion durch Druck zerstäubt und durch warme Luft getrocknet. Für die Zerstäubung der Suspension bei der Trocknung werden Drücke größer 0.1 MPa, bevorzugt größer 2.0 MPa, besonders bevorzugt größer 8.0 MPa angewendet. Die Zerstäubung kann mittels Einstoffdüsen, Zweistoffdüsen oder mittels rotierender Scheiben erfolgen.
Die Trocknung kann bei Zulufttemperaturen von 50 - 200°C vorzugsweise bei 80 - 180°C, erfolgen.

Die erfindungsgemäßen Filmüberzugsmittel können als Pulver oder Granulate mit mittleren Teilchengrößen von 5 µm bis 1000 µm erhalten werden. Die Teilchengröße kann gewünschtenfalls auch durch Mahlprozesse angepasst werden.

Für die Anwendung als Pulver können die erfindungsgemäßen Zubereitungen in Wasser eingerührt, gegebenenfalls mit weiteren Zusätzen, insbesondere farbgebenden Zusätzen, versetzt werden und mittels einer geeigneten Sprüheinrichtung auf das Substrat aufgebracht werden, wobei durch Zufuhr von erwärmter Luft der Filmüberzug sukzessive getrocknet werden kann. Für die Redispergierung der pulverförmigen Filmüberzugsmittel werden üblicherweise keine hochscherenden Rührwerkzeuge benötigt, sondern lediglich einfache langsam drehende Rührer.

Das Aufbringen des Filmüberzugs kann in allen für feste pharmazeutische Darreichungsformen und Nahrungsergänzungsmittel geeigneten Coatingeinrichtungen erfolgen wie z.B. Horizontaltrommelcoatern, Wirbelschichtcoatern, Tauchschwertcoatern, Dragierkesseln.

Für das Zerstäuben der Coatingzubereitung wird vorzugsweise eine Zweistoffdüse verwendet. Die Zulufttemperatur sollte zwischen 20 - 90°C vorzugsweise zwischen 30 - 70°C betragen.

Prinzipiell können alle Kernformen mit gewölbter, konvexer oder konkaver Oberfläche gecoatet werden, unabhängig davon, ob es sich um runde, vieleckige, oblong-Formen oder football-Formen handelt.

Der Kern kann auch ein Subcoating tragen, welches in der Regel aufgebracht wird, um den Wirkstoff zusätzlich zu schützen, z.B. vor Sauerstoff, Protonen oder chemischen Stoffen des Überzuges sowie des Magen- und Darminhaltes.

Auf den erfindungsgemäßen Filmüberzug kann auch ein weiterer Filmüberzug aufgetragen werden, der sich in seiner Zusammensetzung unterscheidet. So kann beispielsweise ein farbloser Filmüberzug oder eine besondere Glanzschicht appliziert werden.

Hinsichtlich der Wirkstoffe gibt es für die erfindungsgemäßen Dosierungsformen keine Beschränkungen. Es können Wirkstoffe aller Indikationsgebiete eingesetzt werden, Humanarzneistoffe wie Tierarzneistoffe, Vitamine, Carotinoide, Nutraceuticals, diätetische Wirkstoffe oder Nahrungsergänzungsmittel, Mineralstoffe, Mikronährstoffe etc. Die Wirkstoffe können unterschiedliche physikochemische Eigenschaften wie Lipophilie, Löslichkeit, Korngröße, Kornstruktur, Oberfläche etc. besitzen.

Die zu überziehenden Darreichungsformen können als Tabletten, Kapseln oder Extrudate vorliegen.

Die erfindungsgemäßen Filmüberzugsmittel weisen verbesserte Eigenschaften hinsichtlich der Wasserdampfpermeation auf. Dies ist besonders im Hinblick auf feuchtigkeitsempfindliche Wirkstoffe von Vorteil.

### Beispiele

Die Prozentangaben beziehen sich, soweit nichts anderes angegeben ist, auf Gew.-%.

### Einsatzstoffe

(A) Komponente A: co-versprühte Mischung aus 60 Gew.-% eines Polyvinylalkohol-Polyethylenglykol-6000-Pfropfpolymers (Gewichtsverhältnis PVA:PEG 75:25, Verseifungsgrad 94 mol-%), 40 Gew.-% Polyvinylalkohol (Mowiol® 5-88, Fa. Kuraray, Viskosität nach DIN 53015 bei 5- 6 mPas) und 0.2 Gew.-% Siliciumdioxid, bezogen auf Gesamtmenge PVA-PEG und PVA, (Kollicoat® Protect, Firma BASF)
(B) Komponente B: Copolymer aus 60 Gew.-% N-Vinylpyrrolidon und 40 Gew.-% Vinylacetat (Copovidon), (Kollidon® VA 64, Firma BASF SE)

### SDS: Natriumlaurylsulfat

### Herstellung der Sprühsuspension - Allgemeine Vorschrift

Die Komponenten A und B wurden in unter Rühren in Wasser aufgelöst, sodass eine 20 gew.-%ige Lösung entstand. Die Pigmente wurden in einem Ultra-Turrax Mischer mit Wasser homogenisiert, sodass eine Pigmentsuspension mit einem Feststoffanteil von 20 Gew.-% erhalten wurde. Anschließend wurde die Pigmentsuspension in die Lösung der Komponenten A und B eingerührt.

**Tabelle: Zusammensetzung bezogen auf Feststoff= 100 Gew.-%**

| Bsp. nr. | Komp. A Gew.-% | Komp. B Gew.-% | Komp. C Pigmente Gew.-% | | SDS Gew.-% | Permeation**) |
|---|---|---|---|---|---|---|
| | | | Talkum | Sicovit Rot *) | | [g µm/m² 24 h] |
| 1 | 18.7 | 6.3 | 67.5 | 7.5 | - | 10459 |
| 2 | 30.0 | 10.0 | 54.0 | 6.0 | - | 9822 |
| 3 | 37.5 | 12.5 | 45.0 | 5.0 | - | 12044 |
| 4 | 45.0 | 15.0 | 36.0 | 4.0 | - | 15171 |
| 5 | 56.2 | 18.8 | 22.5 | 2.5 | - | 19676 |
| 6 | 18.7 | 6.3 | 65.5 | 7.5 | 2.0 | 19143 |
| 7 | 30.0 | 10.0 | 52.0 | 6.0 | 2.0 | 10903 |
| 8 | 37.5 | 12.5 | 43.0 | 5.0 | 2.0 | 12878 |
| 9 | 45.0 | 15.0 | 34.0 | 4.0 | 2.0 | 11020 |
| 10 | 56.2 | 18.8 | 20.5 | 2.5 | 2.0 | 13930 |

| Zum Vergleich | | | | | | |
|---|---|---|---|---|---|---|
| Bsp. nr. | Komp. A Gew.-% | Komp. B Gew.-% | Komp. C Pigmente Gew.-% | | SDS Gew.-% | Permeation |
| | | | Talkum | Sicovit Rot | | [g µm/m² 24 h] |
| i | 25.0 | 0 | 67.5 | 7.5 | 0 | 10742 |
| ii | 40.0 | 0 | 54.0 | 6.0 | 0 | 14847 |
| iii | 50.0 | 0 | 45.0 | 5.0 | 0 | 17247 |
| iv | 60.0 | 0 | 36.0 | 4.0 | 0 | 20769 |
| v | 75.0 | 0 | 22.5 | 2.5 | 0 | 24185 |
| | | | | | | |
| | | | | | | |

| | | | | | | |
|---|---|---|---|---|---|---|
| *) Sicovit® Rot 30 E 172: Eisenoxid, C.I. 77491, Firma BASF SE **) bei 23 °C, 85 % relativer Feuchte, gemessen nach ASTM F-1249. | | | | | | |

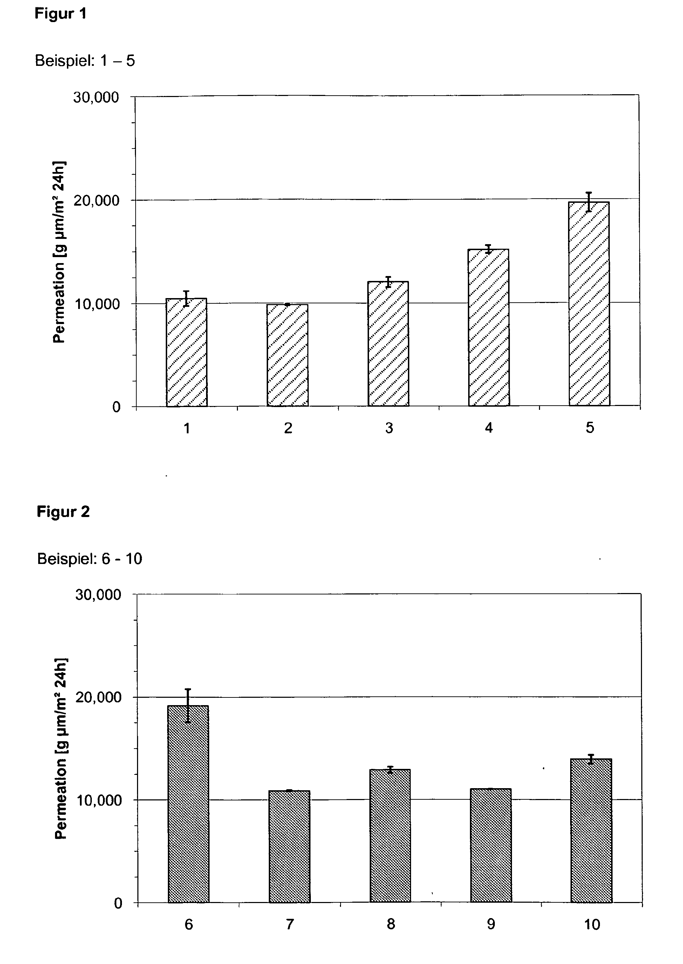

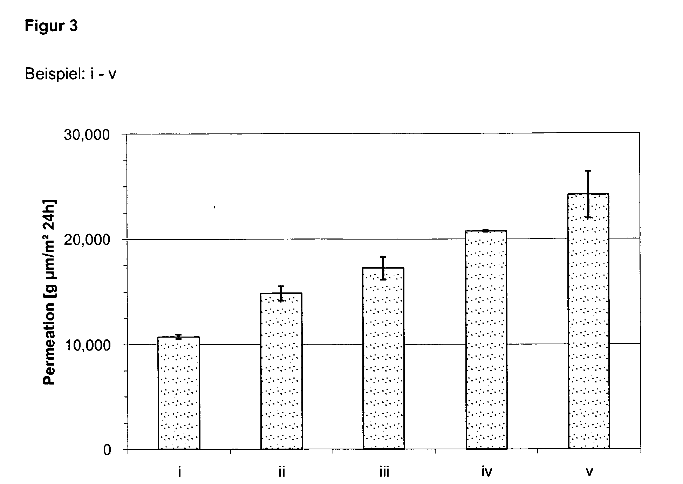

Die Ergebnisse der Permeationsmessungen zur Wasserdampfpermeation sind in den Figuren 1 - 3 graphisch dargestellt.

## Patentansprüche

1. Filmüberzugsmittel basierend auf einer co-prozessierten Mischung aus einem Polyvinylalkohol-Polyether-Pfropfpolymeren und Polyvinylalkohol (Komponente A), einem N-Vinylpyrrolidon-Vinylacetat-Copolymer (Komponente B) und Pigmente (Komponente C), enthaltend
A) 20 - 45 Gew.-% einer co-prozessierten Mischung aus Polyvinylalkohol-Polyether-Pfropfcopolymeren und Polyvinylalkohol (Komponente A),
B) 5 - 20 Gew.-% eines Vinylpyrrolidon-Vinylacetat-Copolymeren (Komponente B)
C) 20 - 75 Gew.-% Pigmente (Komponente C)
D) 0 - 10 Gew.-% eines oder mehrerer Tenside (Komponente D) sowie
E) 0 - 30 Gew.-% weiteren üblichen Coatingbestandteilen (Komponenten E),
und enthaltend als Komponente B ein Copolymer mit einem Gewichtsverhältnis von N-Vinylpyrrolidon zu Vinylacetat von 6:4.

2. Filmüberzugsmittel nach Anspruch 1, enthaltend zusätzlich Tenside (Komponente D)

3. Filmüberzugsmittel nach Anspruch 1 oder 2, enthaltend zusätzlich weitere pharmazeutischen Hilfsstoffe (Komponenten E).

4. Filmüberzugsmittel nach einem der Ansprüche 1 bis 3, enthaltend als Komponente A Polyvinylalkohol-Polyether-Pfropfpolymere, erhalten durch Polymerisation von Vinylacetat in Gegenwart eines Polyethers als Pfropfgrundlage und anschließender Verseifung von mehr als 85 mol-% der Acetatgruppen.

5. Filmüberzugsmittel nach einem der Ansprüche 1 bis 4, enthaltend in der Komponente A einen Polyvinylalkohol mit einem Hydrolysegrad von > 80 mol-%

6. Filmüberzugsmittel nach einem der Ansprüche 1 bis 5, enthaltend eine Komponente A, erhalten durch Versprühen einer gemeinsamen Lösung des Polyvinylalkohol-Polyether-Pfropfpolymers und des Polyvinylalkohols.

7. Filmüberzugsmittel nach einem der Ansprüche 1 bis 6, enthaltend als Komponenten C organische oder anorganische Pigmente.

8. Filmüberzugsmittel nach einem der Ansprüche 1 bis 7, enthaltend als Komponente D Tenside mit einem HLB-Wert größer 10.

9. Filmüberzugsmittel nach einem der Ansprüche 1 bis 8, enthaltend die Komponente D in Mengen von 1 bis 5 Gew.-%.

10. Filmüberzugsmittel nach einem der Ansprüche 1 bis 9, enthaltend die Komponente D Natriumlaurylsulfat.

11. Filmüberzugsmittel nach einem der Ansprüche 1 bis 10, enthaltend als Komponenten E wasserlösliche Farbstoffe, Antiklebemittel, Füllstoffe, Glanzverstärker, Schaumverhinderer, Schutzkolloide, Puffersubstanzen, pH-regulierenden Substanzen oder Haftvermittler oder Gemische davon.

12. Filmüberzugsmittel nach einem der Ansprüche 1 bis11, in Form von Pulvern oder wässrigen Dispersionen.

13. Mit einem Filmüberzug versehene Dosierungsformen, wobei der Filmüberzug durch Aufbringen eines Filmüberzugsmittels gemäß einem der Ansprüche 1 bis 12 erhalten wird.

## Claims

1. A film coating composition based on a co-processed mixture of a polyvinyl alcohol-polyether graft polymer and polyvinyl alcohol (component A), an N-vinylpyrrolidone-vinyl acetate copolymer (component B) and pigments (component C), comprising
A) 20-45% by weight of a co-processed mixture of polyvinyl alcohol-polyether graft copolymers and polyvinyl alcohol (component A),
B) 5-20% by weight of a vinylpyrrolidone-vinyl acetate copolymer (component B)
C) 20-75% by weight of pigments (component C)
D) 0-10% by weight of one or more surfactants (component D) and
E) 0-30% by weight of further customary coating constituents (components E),
and comprising, as component B, a copolymer with a weight ratio of N-vinylpyrrolidone to vinyl acetate of 6:4.

2. The film coating composition according to claim 1, additionally comprising surfactants (component D).

3. The film coating composition according to claim 1 or 2, additionally comprising further pharmaceutical auxiliaries (component E).

4. The film coating composition according to any one of claims 1 to 3, comprising, as component A, polyvinyl alcohol-polyether graft polymers obtained by polymerization of vinyl acetate in the presence of a polyether as graft base and subsequent saponification of more than 85 mol% of the acetate groups.

5. The film coating composition according to any one of claims 1 to 4, comprising, in the component A, a polyvinyl alcohol with a degree of hydrolysis of >80 mol%.

6. The film coating composition according to any one of claims 1 to 5, comprising a component A obtained by spraying a combined solution of the polyvinyl alcohol-polyether graft polymer and of the polyvinyl alcohol.

7. The film coating composition according to any one of claims 1 to 6, comprising, as components C, organic or inorganic pigments.

8. The film coating composition according to any one of claims 1 to 7, comprising, as component D, surfactants with an HLB value greater than 10.

9. The film coating composition according to any one of claims 1 to 8, comprising the component D in amounts of from 1 to 5% by weight.

10. The film coating composition according to any one of claims 1 to 9, comprising the component D sodium lauryl sulfate.

11. The film coating composition according to any one of claims 1 to 10, comprising, as components E, watersoluble dyes, detackifiers, fillers, gloss improvers, foam preventers, protective colloids, buffer substances, pH-regulating substances or adhesion promoters or mixtures thereof.

12. The film coating composition according to any one of claims 1 to 11, in the form of powders or aqueous dispersions.

13. A dosage form provided with a film coating, where the film coating is obtained by applying a film coating composition according to any one of claims 1 to 12.

## Revendications

1. Agent de pelliculage à base d'un mélange cotransformé d'un polymère greffé de poly(alcool vinylique)-polyéther et de poly(alcool vinylique) (composant A), d'un copolymère de N-vinylpyrrolidone-acétate de vinyle (composant B) et de pigments (composant C), contenant
A) 20-45% en poids d'un mélange cotransformé de copolymère greffé de poly(alcool vinylique)-polyéther et de poly(alcool vinylique) (composant A),
B) 5-20% en poids d'un copolymère de vinylpyrrolidone-acétate de vinyle (composant B)
C) 20-75% en poids de pigments (composant C)
D) 0-10% en poids d'un ou de plusieurs agents tensioactifs (composant D) ainsi que
E) 0-30% en poids d'autres constituants usuels de revêtement (composant E)
et contenant, comme composant B, un copolymère présentant un rapport pondéral de N-vinylpyrrolidone à acétate de vinyle de 6:4.

2. Agent de pelliculage selon la revendication 1, contenant en outre des agents tensioactifs (composant D).

3. Agent de pelliculage selon la revendication 1 ou 2, contenant en outre d'autres adjuvants pharmaceutiques (composant E).

4. Agent de pelliculage selon l'une quelconque des revendications 1 à 3, contenant, comme composant A, des polymères greffés de poly(alcool vinylique)-polyéther, obtenus par polymérisation d'acétate de vinyle en présence d'un polyéther comme base de greffage et par saponification consécutive de plus de 85% en mole des groupes acétate.

5. Agent de pelliculage selon l'une quelconque des revendications 1 à 4, contenant, dans le composant A, un poly(alcool vinylique) présentant un degré d'hydrolyse > 80% en mole.

6. Agent de pelliculage selon l'une quelconque des revendications 1 à 5, contenant un composant A obtenu par pulvérisation d'une solution commune du polymère greffé de poly(alcool vinylique)-polyéther et du poly(alcool vinylique).

7. Agent de pelliculage selon l'une quelconque des revendications 1 à 6, contenant, comme composant C, des pigments organiques ou inorganiques.

8. Agent de pelliculage selon l'une quelconque des revendications 1 à 7, contenant, comme composant D, des agents tensioactifs présentant une valeur BHL supérieure à 10.

9. Agent de pelliculage selon l'une quelconque des revendications 1 à 8, contenant le composant D en des quantités de 1 à 5% en poids.

10. Agent de pelliculage selon l'une quelconque des revendications 1 à 9, contenant le composant D laurylsulfate de sodium.

11. Agent de pelliculage selon l'une quelconque des revendications 1 à 10, contenant, comme composant E, des colorants solubles dans l'eau, des agents antiadhésifs, des charges, des agents de renforcement de la brillance, des inhibiteurs de mousse, des colloïdes de protection, des substances tampon, des substances de régulation du pH ou des promoteurs d'adhérence ou des mélanges de ceux-ci.

12. Agent de pelliculage selon l'une quelconque des revendications 1 à 11, sous forme de poudres ou de dispersions aqueuses.

13. Formes de dosage pourvues d'une pellicule, la pellicule étant obtenue par application d'un agent de pelliculage selon l'une quelconque des revendications 1 à 12.
